# EUROPEAN PATENT SPECIFICATION

(11) **EP 1 804 666 B1**
(45) Date of publication and mention of the grant of the patent: **26.08.2015**
(21) Application number: 05807318.0
(22) Date of filing: 05.10.2005
(51) Int. Cl.: A61B 6/00, A61B 8/00, A61B 5/20, A61B 5/145, A61B 5/00

(54) **MEDICAL IMAGING SYSTEM, DISPENSING SYSTEM, METHOD, AND COMPUTER PROGRAM PRODUCT FOR ASSESSING PATIENT RENAL FUNCTION PRIOR TO DISPENSING A CONTRAST MEDIA AS PART OF A MEDICAL IMAGING PROCEDURE**
MEDIZINISCHES DARSTELLUNGSSYSTEM, ABGABESYSTEM, VERFAHREN UND COMPUTERPROGRAMM ZUR BEURTEILUNG DER NIERENFUNKTION EINES PATIENTEN VOR ABGABE EINES KONTRASTMITTELS ALS TEIL EINES MEDIZINISCHEN DARSTELLUNGSVERFAHRENS
SYSTEME D'IMAGERIE MEDICALE, SYSTEME D'ADMINISTRATION, METHODE ET PRODUIT-PROGRAMME INFORMATIQUE POUR L'EVALUATION DE LA FONCTION RENALE D'UN PATIENT AVANT L'ADMINISTRATION D'UN AGENT DE CONTRASTE DANS LE CADRE D'UNE PROCEDURE D'IMAGERIE MEDICALE

(30) Priority: 06.10.2004 US 959466
(43) Date of publication of application: 11.07.2007
(73) Proprietor: ACIST Medical Systems, Inc., Eden Prairie, MN 55344 (US)
(72) Inventor: WILLIAMS, Robert, C., Jr., Fort Salonga, NY 11678 (US); SCHRECK, Brad, Totowa, NJ 07512 (US); CUSHNER, Jeff, Woodmere, NY 11598 (US)
(74) Representative: Jackson, Martin Peter
(86) International application number: PCT/US2005/036113
(87) International publication number: WO 2006/042093

(56) References cited:
- EP-A- 0 364 966
- WO-A-01/37882
- US-A- 2002 028 993
- US-A1- 2002 099 254
- US-A1- 2003 120 171
- US-A1- 2004 044 302

## Description

### FIELD OF THE INVENTION

The present invention relates generally to the analysis of patient biological fluid chemistry prior to a medical imaging procedure that requires the injection of a contrast media. More specifically, the present invention relates to an analysis of biological fluid chemistry risk factors indicating a possible deficiency in renal function in a patient prior to the injection of a contrast media used in a medical imaging procedure. The present invention provides a system and a method, that may be integrated into a medical imaging suite for analyzing biological fluid chemistry risk factors indicating a possible deficiency in renal function in a patient prior to the injection of a contrast media.

### BACKGROUND OF THE INVENTION

Medical imaging procedures often rely on the use of a contrast media that is injected into the biological structure to be imaged such that the medical imaging procedure provides more detailed information to a radiologist or other medical personnel responsible for analyzing the medical imagery. Contrast media is often injected into a patient's vasculature prior to the medical imaging procedure such that the patient's renal system is thereafter tasked with clearing the contrast media from the patient's bloodstream.

According to conventional radiographic diagnostic imaging techniques, such as X-ray procedures, X-rays pass through a target object and expose an underlying photographic film. The developed film then provides an image of the radiodensity pattern of the object. Less radiodense areas produce a greater blackening of the film; more radiodense, bony tissues produce a lighter image. Effective contrast media for X-ray may be either less radiodense than body tissues or more radiodense. The less radiodense agents include air and other gases; an example of a more radiodense contrast material is a barium sulfate suspension or iodinated injectable media.

Computed tomography (CT) is superior to conventional radiography in its ability to image, with extremely high resolution, a succession of thin sections of an object at specific points, lines or planes along the X, Y, or Z axis of the target object. However, because this procedure is also based on the detection of differences in radiodensity, requirements for contrast media in CT are essentially identical with those for conventional radiography.

Magnetic resonance imaging (MRI) systems for body imaging operate on a different physical principle. Generally, MRI relies on the atomic properties (nuclear resonance) of protons in tissues when they are scanned with radio frequency radiation. The protons in the tissue, which resonate at slightly different frequencies, produce a signal that a computer uses to tell one tissue from another. MRI provides detailed three- dimensional soft tissue images.

Fluoroscopy imaging systems may provide real-time X-ray images of internal structures based on differences in the radiodensity of the imaged object components. As in X-ray procedures, fluoroscopy may be enhanced by the use of more radiodense contrast media that may be injected into the object being imaged. For instance, in angiography procedures, radiodense contrast media may be injected into the cardiac vasculature in order to trace the path of blood through the vasculature and determine, for instance, the location of blockages in the cardiac vasculature.

Currently, injection systems used for the dispensing of a contrast media in, for instance, CT, MRI, Ultrasound and/or Angiography/Fluoroscopy medical imaging procedures include interface controls and features limited to the delivery of contrast media within the medical imaging suite. Further, most contrast media is injected to a patient's vasculature for enhancement of imaging procedures and is then physiologically cleared by the renal system through normal nephritic function. During the clearing of contrast media from the patient's body, the serum-borne contrast media places additional burden on renal function until it is cleared. In cases where a patient undergoing a medical imaging procedure using contrast media has a prior history or an unknown pre-existing condition of compromised or impaired renal function, the burden associated with clearing injected contrast media can result in further damage to the kidneys and/or other components of the renal system. Furthermore, in some severe cases, the burden associated with the clearing of iodinated contrast media has destroyed renal function in its totality.

It is possible, however, to perform a blood test whereby blood urea nitrogen (BUN) and creatinine levels can be measured as a method for assessing renal function and a patient's ability to safely clear contrast media. However, current medical imaging systems, such as contrast media injection equipment in existing medical imaging suites, do not provide for the clinical biological fluid chemistry measurements of BUN and creatinine to pre-screen and/or qualify a patient for contrast media injection. In addition, the measurements of BUN and creatinine levels are not made on a substantially real-time basis in the medical imaging suite as part of a medical imaging procedure.

For example, in current inpatient hospital settings, the clinical chemistry laboratory is typically located in a different area of the hospital from the radiology department. As such, either the patient, or a biological fluid sample from the patient must be forwarded to the clinical chemistry laboratory for processing. In the case where a biological fluid sample is transferred to the clinical laboratory, the additional phlebotomist time and expense is incurred. Thereafter, the results must be reported and either transmitted directly to the radiologist from the lab, or indirectly to the radiologist through the referring physician prescribing the radiographic exam in the first place. In short, the logistics of patient routing and transmission of the patient's laboratory results for BUN and creatinine is cumbersome. Similar obstacles are encountered for patients requiring pre-qualifying biological fluid BUN/creatinine analysis prior to undergoing contrast enhanced radiographic examination in an outpatient radiology practice. In this case, the clinical laboratory and radiology office may be in separate buildings separated by large geographic distances.

US 2002/0099254 A1 discloses a method and apparatus to accomplish isolation and removal of a substance from a vasculature by employing a catheter to occlude a vessel of the vasculature. The substance is thus isolated in the vasculature and can be removed.

EP-A-0,364,966 discloses a diagnosis apparatus using an X-ray CT includes an X-ray scanner for repeatedly scanning a single portion of a subject using an X-ray a plurality of times to output X-ray projection data and an injector for injecting a contrast medium into the subject. In the described method blood is repeatedly sampled and analyzed by a bio-chemical analysis to measure a concentration of a contrast medium.

WO-A-01/37882 discloses a method of preventing acute renal failure induced by administration of a radio-contrast agent.

Thus, there exists a need for a medical imaging system, dispensing system, and method for determining, as part of a medical imaging procedure, the presence of biological fluid sample components to assess renal function in a patient scheduled for a medical imaging procedure. There further exists a need for a medical imaging system, dispensing system, and method that may be utilized within a medical imaging suite so that a prospective medical imaging patient may be pre-screened, preferably in real-time, for possible compromised and/or impaired renal function that may be exacerbated by the injection and subsequent clearing of contrast media dispensed to the patient prior to and/or during a medical imaging procedure.

According to one aspect of the present invention there is provided a dispensing system adapted to dispense a contrast media used in a medical imaging procedure, the dispensing system comprising:
a dispensing device configured to dispense the contrast media to a patient;
an analyzing device adapted to receive and analyze a biological fluid sample from the patient so as to determine a level of at least one substance in the biological fluid sample, the analyzing device being adapted to advise an operator of the system of the level of the at least one substance, the analyzing device being further adapted to advise the operator if the level of the at least one substance is within a selected range, and the analyzing device being further configured to communicate with the dispensing device so as to send the level of the at least one substance to the dispensing device,
wherein the at least one substance is selected from the group consisting of:
   blood urea nitrogen;
   creatinine; or
   combinations thereof.

According to another aspect of the invention there is provided a medical imaging system comprising the dispensing system of the first aspect and further comprising a medical imaging device configured to provide an image of a patient using a contrast media dispensed to the patient.

The systems and method of the present invention may aid in the assessment of a patient's renal function prior to the dispensing of a contrast media as part of a medical imaging procedure.

According to other advantageous embodiments the analyzing device may be further configured to communicate either the dispensing device so as to send the level of the at least one substance to the dispensing device. Furthermore, the dispensing device may be further configured to receive the level of the at least one substance and to dispense the contrast media to the patient if the level of the at least one substance is within the selected range. In some embodiments, the medical imaging device, the dispensing device, and the analyzing device may be co-located in a medical imaging suite so as to determine the level of the at least one substance in the medical imaging suite prior to a medical imaging procedure.

In additional embodiments, the analyzing device may further comprise a testing device configured to be in fluid communication with the biological fluid sample such that the testing device may provide a visual indicia to advise the operator of the system of the level of the at least one substance relative to the selected range. In another embodiment, the analyzing device may further comprise a testing device configured to receive the biological fluid sample and to be in fluid communication with the biological fluid sample, and a computer device configured to receive the testing device and to become operably engaged with the testing device to determine the level of the at least one substance in the biological fluid sample.

According to the method aspect of the present invention there is provided a method for assessing renal function of a patient prior to the dispensing of a contrast media as part of a medical imaging procedure, the method comprising the following steps carried out by an analyzing device located in a medical imaging suite:
receiving a biological fluid sample from a patient;
determining a level of at least one substance in the biological fluid sample of the patient;
comparing the level of the at least one substance to a selected range of levels of the at least one substance;
advising an operator of the analyzing device as to whether the level is within the selected range; and
communicating with a dispensing device regarding whether to dispense said contrast media;
wherein the determining step further comprises determining a level of a substance in the biological fluid sample, the substance selected from the group consisting of
   blood urea nitrogen (BUN);
   creatinine; or
   combinations thereof.

As such, the operator may be advised of the patient's renal function prior to dispensing a contrast media without the need to send the patient and/or the biological fluid sample outside of the medical imaging suite for renal function testing.

According to other method embodiments, the determining step may be for the purposes of, for example, assessing patient renal function using quantitative techniques known in the art, such as the calculation of glomerular filtration rate (GFR).

Such embodiments provide significant advantages as described and otherwise discussed herein.

According to another aspect of the invention there is provided a computer program product capable of controlling an analyzing device and a dispensing device located in a medical imaging suite for assessing renal function of a patient prior to the dispensing of a contrast media as part of a medical imaging procedure, the computer program product comprising a computer-readable storage medium having computer-readable program code portions stored therein, the computer-readable program code portions comprising:
an executable portion for determining a level of at least one substance in a biological fluid sample taken from the patient using the analyzing device;
an executable portion for comparing the level of the at least one substance to a selected range of levels of the at least one substance using the analyzing device;
an executable portion for advising an operator of the analyzing device as to whether the level is within the selected range; and
an executable portion for causing the analyzing device to communicate with the dispensing device so as to send the level of the at least one substance to the dispensing device,
wherein the at least one substance is selected from the group consisting of:
   blood urea nitrogen;
   creatinine; or
   combinations thereof.

### BRIEF DESCRIPTION OF THE DRAWINGS

Having thus described the invention in general terms, reference will now be made to the accompanying drawings, which are not necessarily drawn to scale, and wherein:
**FIG. 1** shows one embodiment of the medical imaging system of the present invention wherein the medical imaging device, dispensing device, and analyzer device are co-located within a medical imaging suite;
**FIG. 2** shows one embodiment of the medical imaging system and dispensing system of the present invention wherein the analyzing device is in communication with the dispensing device;
**FIG. 3** shows one embodiment of the medical imaging system of the present invention wherein the analyzing device is in communication with the dispensing device, medical imaging device, and/or a memory device via a network; and
**FIG. 4** shows a medical imaging system and dispensing system wherein the analyzing device comprises a self-contained consumable test strip.

### DETAILED DESCRIPTION OF THE INVENTION

The present inventions now will be described more fully hereinafter with reference to the accompanying drawings, in which some, but not all embodiments of the invention are shown. Indeed, these inventions may be embodied in many different forms and should not be construed as limited to the embodiments set forth herein; rather, these embodiments are provided so that this disclosure will satisfy applicable legal requirements. Like numbers refer to like elements throughout

While the embodiments of the medical imaging system, dispensing system and method for assessing patient renal function prior to a medical imaging procedure are described below in the context of assessing renal function via the determination of a level of at least one substance in a biological fluid sample, it should be understood that the embodiments of the present invention may also be utilized to determine a level and/or the presence of, a variety of substances that may be present in a biological fluid sample so as to assess a patient's ability to safely ingest and/or receive an injection of a contrast media prior to undergoing a medical imaging procedure. The system and method embodiments of the present invention may be used for instance, to provide the capacity to determine a level and/or presence of a variety of substances in a biological sample within, for instance, a medical imaging facility, such that the determination may occur in substantially real time so as to minimize delays that may occur in pre-screening a prospective patient prior to a medical imaging procedure.

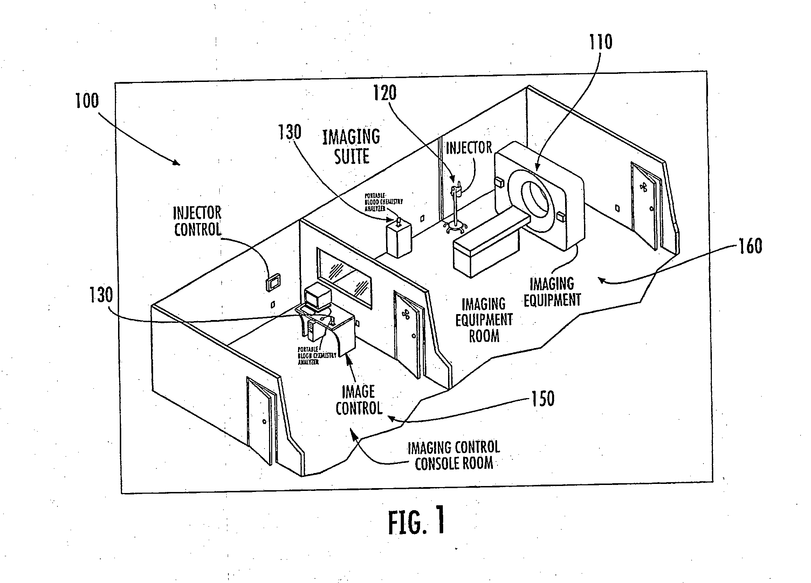

FIG. **1** shows a medical imaging system according to one embodiment of the present invention wherein a medical imaging device **110** is located within a medical imaging suite **100** of a hospital, health care facility, and/or research facility. The medical imaging device of the present invention may comprise, for instance, a computed tomography (CT) scanner, a fluoroscope, a positron emission tomography (PET) scanner, a magnetic resonance (MR) scanner, an ultrasound device and/or other imaging device that may require the dispensing of a contrast media to a patient prior to performing the medical imaging procedure so as to enhance the quality of an image produced by the imaging device **110**. As used herein, the term "medical imaging suite" **100** refers generally to a room or collection of rooms within, for instance, a hospital or other health care facility, wherein various components of a medical imaging system may be located. The medical imaging suite **100** may further comprise, for instance, a control room **150** where an operator of the medical imaging system may be stationed, as well as an imaging room **160** wherein the medical imaging device **110** and other equipment related to a medical imaging procedure may be located. One skilled in the art will appreciate that the medical imaging device **110** may further comprise a computer device operably engaged with the medical imaging device so as to control the operation of the medical imaging device **110** via, for instance, a remotely located controller computer device, that may be located, for instance, in the control room **150** of the medical imaging suite. As such, the medical imaging device **110** may be controlled remotely by an operator of the medical imaging system and the medical imaging device may be further in communication with a computer network via wire connection and/or wireless methods such that images provided by the medical imaging device may be sent to the controller computer device such that the images may be adapted to be viewed by an operator of the medical imaging system and/or stored in a memory device operably engaged with the controller computer device in the control room **150**. As described below, the medical imaging device **110** may further be configured to be in communication with other components of the medical imaging system of the present invention via, for instance, a computer network, such that data related to a given patient and/or medical imaging procedure may be transferred between the components of the medical imaging system of the present invention and/or to other electronic devices connected to or otherwise in communication with the computer network.

FIG. **1** also shows a dispensing device **120** located within the imaging room **160,** for administering contrast media to a patient prior to being subjected to a medical imaging procedure. The dispensing device **120** may be configured to dispense a contrast media that is adapted to be ingested orally by the patient being subjected to the medical imaging procedure, such as, for instance, liquid iodine. The dispensing device **120** may, in some advantageous embodiments, be an injection device, such as, for instance a power injector, configured to inject a contrast media directly into the vasculature of the patient prior to the inception of the medical imaging procedure. In some embodiments, the dispensing device **120** may further comprise a computer device operably engaged therewith, wherein the computer device may be configured to be connected via wire connection or wireless methods to a computer network. Thus, the dispensing device **120** may be controlled remotely by an operator of the medical imaging system by, for instance, a controller computer device, configured to communicate via the computer network, with the dispensing device **120** such that he dispensing device **120** may be located in the imaging room **160** while the operator of the medical imaging system may control the dispensing device **120** from, for instance, a control room **150** adjacent to the imaging room **160** or located elsewhere within the medical imaging suite **100**.

Also shown in FIG. **1** is an analyzing device **130**, co-located with the medical imaging device **110**, and the dispensing device **120**, within the medical imaging suite **100**. The analyzing device **130**, according to the present invention, is adapted to receive and analyze a biological fluid sample from the patient so as to determine a level of at least one substance in the biological fluid sample prior to the dispensing of contrast media by, for instance, the dispensing device **120**. The biological fluid sample may comprise, for instance, a blood sample, urine sample, saliva sample, and/or other biological fluid samples suitable for analysis in the analysing device **130**. The analyzing device **130** is further adapted to advise an operator of the system of the level of the at least one substance, and to advise the operator to dispense the contrast media (via, for instance, the dispensing device **120** or alternatively imaging device **110**), if the level of the at least on substance is within a selected range and/or above or below a selected threshold level. As such, the analyzing device **130** may provide for a substantially real-time determination of the level of the at least one substance so as to allow the operator of the medical imaging system (and/or other medical personnel) to assess, for instance, the ability of the patient to safely be injected with the contrast media, prior to the inception of the medical imaging procedure. The analyzing device **130** of the present invention may determine a level of blood urea nitrogen (BUN) and/or creatinine in a blood sample taken from a prospective patient so as to assess the ability of the prospective patient to safely clear the contrast media from their vascular system without causing damage to the renal system of the prospective patient. One skilled in the art will appreciate that determination of BUN and/or creatinine levels may allow medical personnel to assess the prospective patient's renal function and thereby preliminarily determine the prospective patient's ability to clear dispensed contrast media via the patient's renal system. Such assessments may be, in some examples, accomplished via the calculation of glomerular filtration rate (GFR) using measured creatinine levels and patient data. The analyzing device may, however, be further configured to detect and/or determine a level of a variety of substances within a biological fluid sample taken from a prospective patient so as to assess the patient's suitability to be subjected to a particular type of medical imaging procedure without requiring the patient or a biological fluid sample associated with the patient to be sent outside of the medical imaging suite **100.**

As shown in FIG. **1**, the analyzing device **130** of the present invention may, in some embodiments, also be located in a control room **150** of the medical imaging suite **100** such that an operator of the medical imaging system may obtain a biological fluid sample from a prospective patient located, for instance in the imaging room **160** and subsequently bring the biological fluid sample into contact with the analyzing device **130** within the control room **150** so as to determine a level of at least one substance in the biological fluid sample prior to initiating the dispensing of contrast media. As described above, the analyzing device **130** may be further adapted to advise the operator to dispense the contrast media (via, for instance, the dispensing device **120** or alternatively imaging device **110**), if the level of the at least one substance is within a selected range. The selected range may, for instance, be indicative of a range of levels of the at least one substance indicating that the patient has a substantially normal renal function which would allow the patient to safely clear the contrast media from their bloodstream. According to this embodiment, if the level of the at least one substance is within the selected range, the operator may then remotely initiate the medical imaging procedure from the control room **150**, by for instance, remotely controlling the dispensing device **120** to dispense the contrast media to the patient and subsequently remotely controlling the medical imaging device **110** to provide an image of the patient. This embodiment may be suitable for minimizing unnecessary radiation exposure to the operator if, for instance, the medical imaging procedure utilizes radioactive emissions to provide an image, and/or in embodiments wherein the contrast media to be dispensed comprises a radioactive substance.

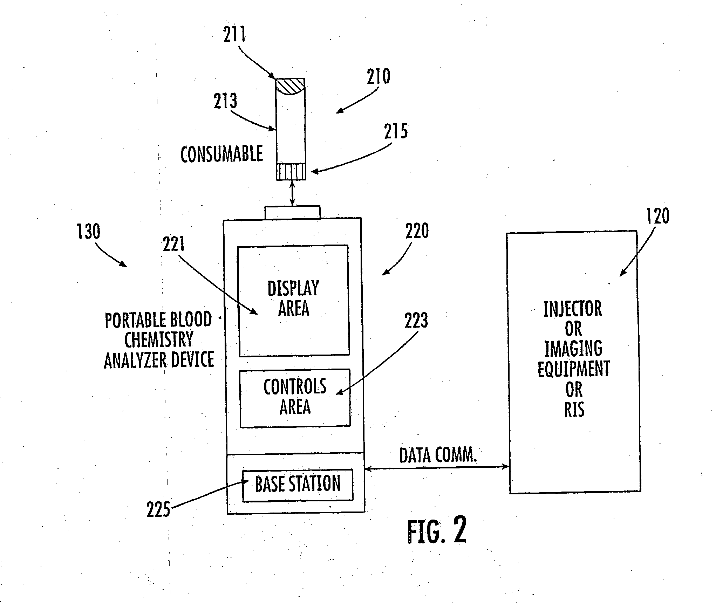

FIG. **2** shows a schematic representation of the analyzing device **130** according to one embodiment of the present invention. As shown, the analyzing device may further comprise a testing device **210** configured to receive and be in fluid communication with a biological fluid sample taken from a prospective patient and a computer device **220** configured to receive the testing device **210** and to become operably engaged therewith to determine the level of the at least one substance in the biological fluid sample. The testing device **210** may further comprise, for instance, a biological fluid sample collection reservoir **211**, at least one reagent **213** configured to interact with the at least one substance, and a connecting (this may be a better choice of words from a technical standpoint) device **215** configured to communicate with the computer device **220** such that the computer device **220** may further determine the level of the at least one substance in the biological fluid sample. The biological fluid sample collection reservoir may further comprise, for instance, a plurality of capillaries configured to receive the biological fluid sample and transfer, via, for instance, capillary action, the biological sample to a portion of the biological fluid sample collection reservoir **211** containing at least one reagent **213** or other biochemical material suitable for reacting with the biological fluid sample for the purposes of determining a level of the at least one substance. For instance, in some embodiments, the reagent **213** may react with a biological fluid sample to produce a color change, and/or ionization, and/or electro-chemical reaction within the testing device **210** such that the connecting device **215** may transmit, for instance, the degree of color change, and/or ionization, and/or electro-chemical reaction occurring within the testing device to a computer device **220** (as described more fully below) via for instance, electrical, and/or electro-optical, and/or electro-chemical methods such that the computer device **220** may determine the level of the at least one substance in the biological fluid sample. According to some embodiments, the testing device **210** may comprise a consumable test strip further comprising the biological fluid sample collection reservoir **211**, reagent **213**, and connecting device **215** as described above such that each testing device **210** may be discarded after determining the level of the at least one substance in a given biological fluid sample. Thus, a new testing consumable test strip may be used for analyzing a biological fluid sample from each prospective patient entering the medical imaging suite **100**.

Also, as shown in FIG. **2**, the analyzing device **130** may further comprise a computer device **220**, as described generally above, which may be re-used and configured to receive a testing device **210** corresponding to a biological fluid sample related to each prospective patient entering the medical imaging suite **100**. The computer device **220** may be further configured to communicate with the testing device **210** via, for instance, the transmitting device **215** operably engaged with the testing device **210**. As described above, the computer device may communicate with the testing device **210** via electrical, and/or electro-optical, and/or electro-chemical methods so as to determine a degree of reaction of the reagent **213** with the biological fluid sample so as to determine a level of the at least one substance in the biological fluid sample. Also, as shown in FIG. **2**, the computer device **220** may further comprise a display **221** and an input device **223**. The computer device **220** may further comprise a memory device such that an operator of the medical imaging system may enter, via, for instance, the input device **223** data related to the medical imaging procedure, including patient information, the selected range of the level of the at least one substance, and/or other information related to the medical imaging procedure. Thus, the computer device **220** may compare the level of the at least one substance in the biological fluid sample as determined by the testing device **210** with the selected range entered by an operator of the medical imaging system, so as to advise the operator of the medical imaging system, via, for instance, the display **221**, whether or not the operator may safely dispense the contrast media to the patient. The computer device **220** may comprise a variety of electronic devices including, for instance, a personal computer (including laptop personal computers), a PDA, palmtop computer devices, and/or other computer devices suitable for operable engagement with the base station **225** and/or the testing device **210**. In addition, the display **221** may comprise, for instance, a cathode ray tube (CRT), LCD, LCD touch-screen, or other display device suitable for displaying text, images, graphics, and/or numerical data related to the medical imaging procedure and the level of the at least one substance in the biological fluid sample relative to the selected range input by, for instance, an operator of the medical imaging system.

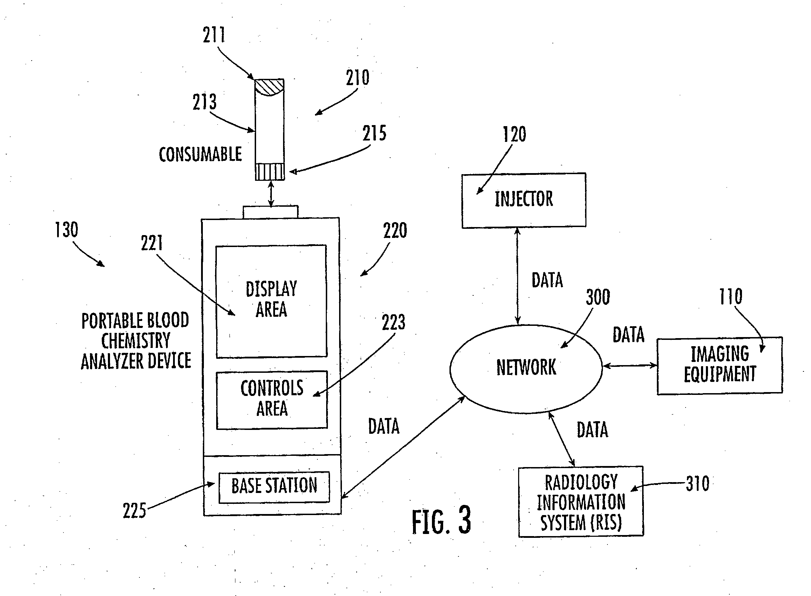

As shown in FIGS. **2** and **3**, the computer device **220** may be configured to become operably engaged with a base station **225** such that the computer device **220** may be removed from the base station and carried by, for instance, an operator of the medical imaging system. Thus, in this embodiment, the computer device may be carried by an operator of the medical imaging system so as to allow the operator to obtain a biological fluid sample from the prospective patient and determine a level of the at least one substance in the biological fluid sample in substantially real time within the medical imaging suite **100**. The operator may then return the computer device to an operable engagement with the base station **225**. According to other advantageous embodiments, the base station may be a wireless network node, such that the computer device may remain in communication with the base station even as the computer device **220** is carried throughout the medical imaging suite **100.** The base station **225** may also comprise various types of network devices, such as a network node and/or router, such that when the computer device **220** becomes operably engaged with the base station **225,** the computer device **220** may be further configured to communicate with the computer network **300.** As shown in FIG. 3, the computer device **220** may be also configured to be in communication with the medical imaging device **110** and/or the dispensing device **120** via, for instance, the computer network **300**. Furthermore, the analyzing device **130** (and associated computer device **220**) is further configured to be in communication (via, for instance, the computer network **300**) with the dispensing device **120** so as to be capable of sending the level of the at least one substance to the dispensing device **120**. Furthermore, the dispensing device **120** may be configured to receive the level of the at least one substance and to dispense the contrast media to the patient if the level of the at least one substance is within the selected range. This embodiment may also provide for an operator lock-out feature such that if, for instance, the operator attempts to dispense a contrast media and/or initiate a medical imaging procedure wherein the determined level of the at least one substance is outside of the selected range (which may indicate that the prospective patient is not suited to receive the contrast media) the analyzing device **130** may send a lock-out signal to the dispensing device **120** such that the operator may not dispense the contrast media before entering an override code. The lock-out feature may be accomplished for instance, by sending an electronic signal, via the computer network **300** from the computer device **220** to the dispensing device **120** (or a computer device operably engaged therewith) that the level of the at least one substance in the biological fluid sample is outside the selected range corresponding to a patient's ability to safely clear a given contrast media. According to other embodiments of the present invention, an electronic signal may also be sent, via the computer network **300**, from the computer device **220** to the medical imaging device **110** to lock-out an operator of the medical imaging system when the level of the at least one substance in the biological fluid sample is outside the selected range corresponding to a patient's ability to safely clear a given contrast media. These lock-out features may provide an additional safety feature to some embodiments in order to prevent the dispensing of contrast media to a prospective patient exhibiting levels of the at least one substance outside of the selected range, which may, in turn, indicate that the patient may have difficulties in safely clearing contrast media from their bloodstream via, for instance, the renal system.

In some embodiments, the medical imaging system of the present invention may further comprise a database **310** configured to store data related to individual patient histories, the level of the at least one substance in the biological fluid sample for past screenings of patients, as well as storing selected range data suitable for screening for substantially normal renal function and/or other physiological information pertinent to assessing a prospective patient's eligibility to receive a contrast media as part of a medical imaging procedure. One skilled in the art will appreciate that such data may include patient data (such as height, weight, race/ethnicity, sex, age, and other patient characteristics) used in combination with a determined level of creatinine to calculate a glomerular filtration rate (GFR) for a particular patient. The database **310** may be stored in a memory associated with a computer device wherein the computer device may be in communication with the computer network **300** as shown in FIG. 3. Thus, the database **310** may be interrogated by, for instance, the imaging device **110,** dispensing device **120,** and/or analyzing device **130** such that an operator of the medical imaging system and dispensing system of the present invention may gain access to the data stored in the database **310**. Thus, in some cases, wherein for instance, a patient must undergo multiple medical imaging procedures, the dispensing device **120** may interrogate the database **310** to determine the level of the at least one substance in a biological fluid sample taken from the patient prior to a first medical imaging procedure. In addition, the database may be interrogated by medical professionals seeking patient history related to, for instance, the patient's renal function, and/or history of medical imaging procedures.

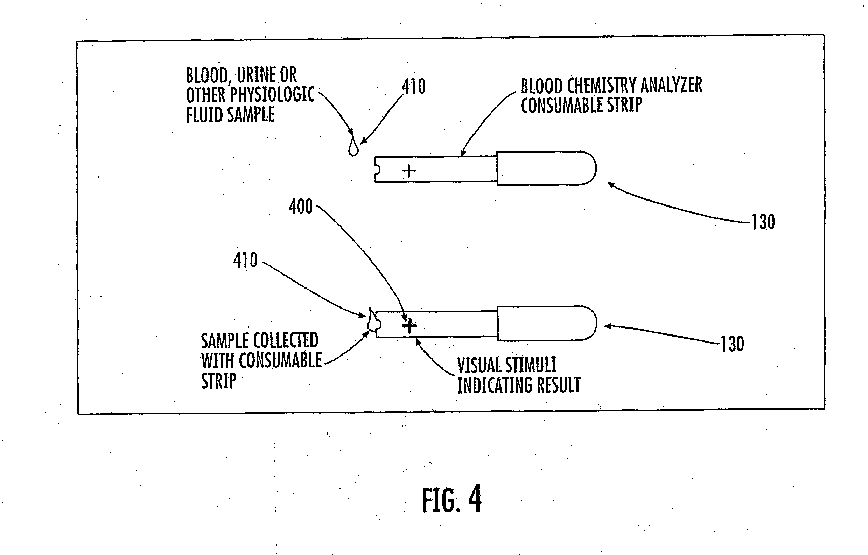

As shown in FIG. **4**, an analyzing device **130** of a medical imaging system may alternatively comprise a self-contained consumable testing device **130** configured to be in fluid communication with a biological fluid sample **410**. Furthermore, the testing device **130** may be further configured to provide a visual indicia **400** to advise the operator of the system of the level of the at least one substance relative to the selected range. The self-contained consumable testing device **130** may further comprise a capillary configured to receive the biological fluid sample **410** from a prospective patient. The self-contained consumable testing device may further comprise a reagent adapted to react with at least one substance in the biological fluid sample **410** such that the reagent may produce a visual indicia **400**, such as for instance, a color change, and/or a symbolic indicia to indicate that the level of at least one substance is within a selected range such that a contrast media may be safely dispensed to the patient in conjunction with a medical imaging procedure. According to this medical imaging system, a plurality of self-contained consumable testing devices **130** may be made available in the medical imaging suite **100** such that an operator of the medical imaging system may quickly determine, via the self-contained consumable testing device **130**, if a particular patient may be eligible to safely receive an administration of a contrast media used in a medical imaging procedure. The medical imaging system and/or dispensing system comprising a self-contained consumable testing device **130** (as shown in FIG. **4**) may be preferable for use in hospitals wherein existing medical imaging suites exist having medical imaging devices **110** and/or dispensing devices **120** that are non-network capable, or where cost restrictions prevent the purchase of a computer device-based analyzing device **130**.

The present invention also provides method embodiments for assessing the renal function of a patient prior to the dispensing of a contrast media as part of a medical imaging procedure such that the assessment may occur without sending the prospective patient and/or a biological fluid sample associated with the prospective patient outside the medical imaging suite **100**. According to one embodiment, the method comprises the steps of: determining a level of at least one substance in the biological fluid sample of the patient using an analyzing device **130** located in a medical imaging suite **100**; comparing the level of the at least one substance to a selected range of levels of the at least one substance using the analyzing device **130** located in the medical imaging suite **100**; and advising an operator of the analyzing device as to whether the level is within the selected range such that the operator may be advised of the patient's renal function prior to dispensing a contrast media as part of a medical imaging procedure.

According to other method examples not part of the invention, the method may further comprise the step of dispensing the contrast media to the patient using a dispensing device **120** if the level of the at least one substance is within the selected range. As such, this example of the method may pre-screen the patient for a substantially normal renal function prior to dispensing the contrast media via the dispensing device **120**.
According to the invention, the determining step further comprises determining a level of blood urea nitrogen (BUN), creatinine, or combinations thereof in the biological fluid sample.

The present invention also provides computer program product embodiments capable of executing the various method steps of the present invention. According to some embodiments, the computer program product may be executable on the computer device **220**, dispensing device **120**, and/or imaging device **110**. The computer program embodiments of the present invention may be further configured to receive patient physiological data including, but not limited to parameters such as height, weight, sex, age, pre-existing medical conditions, patient-identifier information, and other data that may be relevant to the medical imaging procedure. Such data may also, in some embodiments, include other information such as time, date, location of medical imaging procedure, lot numbers for various pharmaceuticals, contrast media, or other medical supplies used in the medical imaging procedure, and/or other data related to the medical imaging procedure.

According to some embodiments, the data described above may be received by the computer device **220**, dispensing device **120**, and/or imaging device **110** via the computer program product embodiments from the database **310** or from a user interface, such as a keyboard, mouse, touch screen or other user interface that may be operably engaged with and/or in communication with (via wire or wireless methods) the computer device **220,** dispensing device **120,** and/or imaging device **110.** The computer program product embodiments may also be configured to receive the level of the at least one substance (such as, for instance, blood urea nitrogen (BUN), creatinine, or combinations thereof) in the biological fluid sample that may be determined by the analyzing device **130** and determine, for instance, based on the received data, if an alternate volume, type, concentration, and/or combination of one or more contrast media may be properly and safely administered to the patient by the dispensing device **120** such that the contrast media may be safely cleared by the renal function of the patient.

Many modifications and other embodiments of the invention will come to mind to one skilled in the art to which this invention pertains having the benefit of the teachings presented in the foregoing descriptions and the associated drawings. For example, one skilled in the art will appreciate that the systems, methods, and computer program products disclosed herein may also be used to determine a level of at least one substance in the biological fluid sample so as to enable the further determination of a corresponding volume, type, concentration, and/or combination of one or more contrast media that may be properly and safely administered to a patient such that the contrast media may be safely cleared by the renal function of the patient. Therefore, it is to be understood that the invention is not to be limited to the specific embodiments disclosed and that modifications and other embodiments are intended to be included within the scope of the appended claims. Although specific terms are employed herein, they are used in a generic and descriptive sense only and not for purposes of limitation.

## Claims

1. A dispensing system adapted to dispense a contrast media used in a medical imaging procedure, the dispensing system comprising:
a dispensing device (120) configured to dispense the contrast media to a patient;
an analyzing device (130) adapted to receive and analyze a biological fluid sample from the patient so as to determine a level of at least one substance in the biological fluid sample, the analyzing device (130) being adapted to advise an operator of the system of the level of the at least one substance, the analyzing device (130) being further adapted to advise the operator if the level of the at least one substance is within a selected range, and the analyzing device (130) being further adapted to be in communication with the dispensing device (120) regarding whether to dispense the contrast media;
wherein the at least one substance is selected from the group consisting of:
blood urea nitrogen (BUN);
creatinine; or
combinations thereof.

2. A dispensing system according to Claim 1, wherein the analyzing device (130) is further adapted to advise the operator to dispense the contrast media if the level of the at least one substance is within the selected range.

3. A dispensing system according to Claim 1, wherein the analyzing device (130) is further configured to cooperate with the dispensing device (120) such that the dispensing device (120) is further configured to dispense the contrast media to the patient if the level of the at least one substance sent by the analysing device (130) is within the selected range.

4. A dispensing system according to Claim 1, wherein the analyzing device (130) further comprises a testing device (210) configured to be in fluid communication with the biological fluid sample such that the testing device (210) is further configured to provide a visual indicia (400) to advise the operator of the system of the level of the at least one substance relative to the selected range.

5. A dispensing system according to Claim 1, wherein the analyzing device (130) further comprises:
a testing device (210) configured to receive the biological fluid sample and to be in fluid communication therewith; and
a computer device (220) configured to receive the testing device (210) and to become operably engaged therewith to determine the level of the at least one substance in the biological fluid sample.

6. A dispensing system according to Claim 5, wherein the testing device (210) further comprises a biological fluid sample reservoir (211), at least one reagent configured to interact with the at least one substance and a transmitting device (215) configured to communicate with the computer device (220), and wherein the computer device (220) is further configured to communicate with the testing device (210) so as to determine the level of the at least one substance.

7. A dispensing system according to Claim 6, wherein the computer device (220) further comprises a display (221) configured to provide visual indicia so as to advise the operator of the system of the level of the at least one substance.

8. A dispensing system according to Claim 6, wherein the computer device (220) further comprises an input device (223) configured to receive data selected from the group consisting of:
data related to the patient;
data related to the selected range;
and combinations thereof.

9. A medical imaging system comprising the dispensing system according to claims 1, 2 or 4-8, and further comprising:
a medical imaging device (110) configured to provide an image of the patient using a contrast media dispensed to the patient.

10. A medical imaging system according to Claim 9 when dependent upon claim 1, and wherein the dispensing device (120) is further configured to dispense the contrast media to the patient if the level of the at least one substance sent by the analyzing device (130) is within the selected range.

11. A medical imaging system according to Claim 9 when dependent upon Claim 5, wherein the computer device (220) is configured to be capable of calculating a glomerular filtration rate using the received data and the level of the at least one substance.

12. A method for assessing renal function of a patient prior to the dispensing of a contrast media as part of a medical imaging procedure, the method comprising the following steps carried out by an analyzing device (130) located in a medical imaging suite (100):
receiving a biological fluid sample from a patient;
determining a level of at least one substance in the biological fluid sample of the patient;
comparing the level of the at least one substance to a selected range of levels of the at least one substance;
advising an operator of the analyzing device (130) as to whether the level is within the selected range; and
communicating with a dispensing device regarding whether to dispense said contrast media;
wherein the determining step further comprises determining a level of a substance in the biological fluid sample, the substance selected from the group consisting of:
blood urea nitrogen (BUN);
creatinine; or
combinations thereof.

13. A computer program product capable of controlling an analyzing device (130) and a dispensing device (120) located in a medical imaging suite (100) for assessing renal function of a patient prior to the dispensing of a contrast media as part of a medical imaging procedure, the computer program product comprising a computer-readable storage medium having computer-readable program code portions stored therein, the computer-readable program code portions comprising:
an executable portion for determining a level of at least one substance in a biological fluid sample taken from the patient using the analyzing device (130);
an executable portion for comparing the level of the at least one substance to a selected range of levels of the at least one substance using the analyzing device (130);
an executable portion for advising an operator of the analyzing device as to whether the level is within the selected range; and
an executable portion for causing said analyzing device (130) to communicate with said dispensing device regarding whether to dispense said contrast media;
wherein the at least one substance is selected from the group consisting of:
blood urea nitrogen (BUN);
creatinine; or
combinations thereof.

14. A computer program product according to Claim 13, further comprising an executable portion for dispensing the contrast media to the patient using the dispensing device (120) if the level of the at least one substance is within the selected range such that the patient is screened for substantially normal renal function prior to dispensing the contrast media.

15. A computer program product according to Claim 13, further comprising an executable portion for adjusting a property of the contrast media using the dispensing device (120) to create an altered contrast media if the level of the at least one substance is outside the selected range such that the altered contrast media may be cleared by the renal function of the patient after dispensing the altered contrast media.

16. A computer program product according to Claim 15 wherein the property of the contrast media is selected from the group consisting of:
volume of the contrast media;
delivery rate of the contrast media;
concentration of the contrast media;
type of the contrast media; or
combinations thereof.

## Patentansprüche

1. Abgabesystem zur Abgabe eines Kontrastmittels, das in einem medizinischen Darstellungsverfahren verwendet wird, wobei das Abgabesystem Folgendes umfasst:
eine Abgabevorrichtung (120), die zur Abgabe des Kontrastmittels an einen Patienten ausgelegt ist;
eine Analysevorrichtung (130), die ausgelegt ist, eine biologische Flüssigkeitsprobe von dem Patienten zu erhalten und zu untersuchen, um eine Konzentration zumindest einer Substanz in der biologischen Flüssigkeitsprobe zu bestimmen, wobei die Analysevorrichtung (130) ausgelegt ist, eine Bedienungsperson des Systems über die Konzentration der zumindest einen Substanz zu informieren, wobei die Analysevorrichtung (130) ferner ausgelegt ist, die Bedienungsperson zu informieren, wenn die Konzentration der zumindest einen Substanz innerhalb eines gewählten Bereichs liegt, und wobei die Analysevorrichtung (130) ferner ausgelegt ist, mit der Abgabevorrichtung (120) in Bezug darauf, ob das Kontrastmittel abgegeben werden soll, zu kommunizieren;
worin die zumindest eine Substanz aus der aus:
Blut-Harnstoff-Stickstoff (BUN);
Creatinin; oder
Kombinationen davon bestehenden Gruppe ausgewählt ist.

2. Abgabesystem nach Anspruch 1, worin die Analysevorrichtung (130) ferner ausgelegt ist, die Bedienungsperson zu informieren, dass das Kontrastmittel abgegeben werden soll, wenn die Konzentration der zumindest einen Substanz innerhalb des gewählten Bereichs liegt.

3. Abgabesystem nach Anspruch 1, worin die Analysevorrichtung (130) ferner ausgelegt ist, mit der Abgabevorrichtung (120) zusammenzuarbeiten, so dass die Abgabevorrichtung (120) ferner ausgelegt ist, das Kontrastmittel an den Patienten abzugeben, wenn die von der Analysevorrichtung (130) gesendete Konzentration der zumindest einen Substanz innerhalb des gewählten Bereichs liegt.

4. Abgabesystem nach Anspruch 1, worin die Analysevorrichtung (130) ferner eine Testvorrichtung (210) umfasst, die ausgelegt ist, mit der biologischen Flüssigkeitsprobe in Fluidverbindung zu stehen, so dass die Testvorrichtung (210) ferner ausgelegt ist, einen visuellen Hinweis (400) bereitzustellen, um die Bedienungsperson des Systems über die Konzentration der zumindest einen Substanz im Verhältnis zu dem gewählten Bereich zu informieren.

5. Abgabesystem nach Anspruch 1, worin die Analysevorrichtung (130) ferner Folgendes umfasst:
eine Testvorrichtung (210), die zum Erhalten der biologischen Flüssigkeitsprobe und zur Fluidverbindung damit ausgelegt ist; und
eine Computervorrichtung (220), die ausgelegt ist, die Testvorrichtung (210) aufzunehmen und mit dieser in Wirkeingriff gesetzt zu werden, um die Konzentration der zumindest einen Substanz in der biologischen Flüssigkeitsprobe zu bestimmen.

6. Abgabesystem nach Anspruch 5, worin die Testvorrichtung (210) ferner ein Reservoir (211) für biologische Flüssigkeitsproben, zumindest ein Reagenz, das zur Wechselwirkung mit der zumindest einen Substanz ausgelegt ist, und eine zur Verbindung mit der Computervorrichtung (220) ausgelegte Übertragungsvorrichtung (215) umfasst, worin die Computervorrichtung (220) ferner zur Kommunikation mit der Testvorrichtung (210) zur Bestimmung der Konzentration der zumindest einen Substanz ausgelegt ist.

7. Abgabesystem nach Anspruch 6, worin die Computervorrichtung (220) ferner ein Display (221) umfasst, das zur Bereitstellung von visuellen Hinweisen ausgelegt ist, um die Bedienungsperson des Systems über die Konzentration der zumindest einen Substanz zu informieren.

8. Abgabesystem nach Anspruch 6, worin die Computervorrichtung (220) ferner eine Eingabevorrichtung (223) umfasst, die zum Empfangen von Daten ausgelegt ist, die aus der aus:
Daten in Bezug auf den Patienten;
Daten in Bezug auf den gewählten Bereich;
und Kombinationen davon bestehenden Gruppe ausgewählt sind.

9. Medizinisches Darstellungssystem, das Abgabesystem nach den Ansprüchen 1, 2 oder 4-8 umfassend, und ferner Folgendes umfassend:
eine medizinische Bildgebungsvorrichtung (110), die zur Bereitstellung eines Bildes des Patienten unter Verwendung eines an den Patienten abgegebenen Kontrastmittels ausgelegt ist.

10. Medizinisches Darstellungssystem nach Anspruch 9, in Abhängigkeit von Anspruch 1, und worin die Abgabevorrichtung (120) ferner ausgelegt ist, das Kontrastmittel an den Patienten abzugeben, wenn die von der Analysevorrichtung (130) gesendete Konzentration der zumindest einen Substanz innerhalb des gewählten Bereichs liegt.

11. Medizinisches Darstellungssystem nach Anspruch 9, in Abhängigkeit von Anspruch 5, worin die Computervorrichtung (220) ausgelegt ist, eine glomeruläre Filtrationsrate unter Verwendung der empfangenen Daten und der Konzentration der zumindest einen Substanz berechnen zu können.

12. Verfahren zur Beurteilung der Nierenfunktion eines Patienten vor Abgabe eines Kontrastmittels als Teil eines medizinischen Darstellungsverfahrens, wobei das Verfahren die folgenden Schritte umfasst, die von einer Analysevorrichtung (130) ausgeführt werden, die sich in einer medizinischen Darstellungs-Suite (100) befindet:
Erhalten einer biologischen Flüssigkeitsprobe von einem Patienten;
Bestimmen einer Konzentration zumindest einer Substanz in der biologischen Flüssigkeitsprobe des Patienten;
Vergleich der Konzentration der zumindest einen Substanz mit einem gewählten Bereich von Konzentrationen der zumindest einen Substanz;
Informieren einer Bedienungsperson der Analysevorrichtung (130), ob die Konzentration innerhalb des gewählten Bereichs liegt; und
Kommunizieren mit einer Abgabevorrichtung in Bezug darauf, ob das Kontrastmittel abgegeben werden soll;
worin der Schritt des Bestimmens ferner die Bestimmung einer Konzentration einer Substanz in der biologischen Flüssigkeitsprobe umfasst, wobei die Substanz aus der aus:
Blut-Harnstoff-Stickstoff (BUN);
Creatinin; oder
Kombinationen davon bestehenden Gruppe ausgewählt ist.

13. Computerprogrammprodukt, das zur Steuerung einer Analysevorrichtung (130) und einer Abgabevorrichtung (120) in einer medizinischen Darstellungs-Suite (100) zur Beurteilung der Nierenfunktion eines Patienten vor der Abgabe eines Kontrastmittels als Teil eines medizinischen Darstellungsverfahrens befähigt ist, wobei das Computerprogrammprodukt ein computerlesbares Speichermedium mit darin gespeicherten computerlesbaren Programmcodeteilen umfasst, wobei die computerlesbaren Programmcodeteile Folgendes umfassen:
einen ausführbaren Teil zur Bestimmung einer Konzentration zumindest einer Substanz in einer biologischen Flüssigkeitsprobe, die dem Patienten entnommen wurde, unter Verwendung der Analysevorrichtung (130);
einen ausführbaren Teil zum Vergleichen der Konzentration der zumindest einen Substanz mit einem gewählten Bereich von Konzentrationen der zumindest einen Substanz unter Verwendung der Analysevorrichtung (130) ;
einen ausführbaren Teil zur Information einer Bedienungsperson der Analysevorrichtung, ob die Konzentration innerhalb des gewählten Bereichs liegt; und
einen ausführbaren Teil zum Veranlassen der Analysevorrichtung (130), mit der Abgabevorrichtung in Bezug darauf zu kommunizieren, ob Kontrastmittel abgegeben werden soll;
worin die zumindest eine Substanz aus der aus:
Blut-Harnstoff-Stickstoff (BUN);
Creatinin; oder
Kombinationen davon bestehenden Gruppe ausgewählt ist.

14. Computerprogrammprodukt nach Anspruch 13, ferner umfassend einen ausführbaren Teil zur Abgabe des Kontrastmittels an den Patienten unter Verwendung der Abgabevorrichtung (120), wenn die Konzentration der zumindest einen Substanz innerhalb des gewählten Bereichs liegt, so dass der Patient vor Abgabe des Kontrastmittels auf eine im Wesentlichen normale Nierenfunktion untersucht wird.

15. Computerprogrammprodukt nach Anspruch 13, ferner umfassend einen ausführbaren Teil zum Einstellung einer Eigenschaft des Kontrastmittels unter Verwendung der Abgabevorrichtung (120) zur Erzeugung eines veränderten Kontrastmittels, wenn die Konzentration der zumindest einen Substanz außerhalb des gewählten Bereichs liegt, so dass das veränderte Kontrastmittel nach Abgabe des veränderten Kontrastmittels durch die Nierenfunktion des Patienten ausgeschieden werden kann.

16. Computerprogrammprodukt nach Anspruch 15, worin die Eigenschaft des Kontrastmittels aus der aus:
Volumen des Kontrastmittels;
Abgabegeschwindigkeit des Kontrastmittels;
Konzentration des Kontrastmittels;
Art des Kontrastmittels; oder
Kombinationen davon bestehenden Gruppe ausgewählt ist.

## Revendications

1. Système d'administration adapté pour administrer un agent de contraste utilisé dans une procédure d'imagerie médicale, le système d'administration comprenant:
un dispositif d'administration (120) qui est configuré pour administrer l'agent de contraste à un patient;
un dispositif d'analyse (130) qui est adapté pour recevoir et analyser un échantillon de fluide biologique provenant du patient de manière à déterminer un niveau d'au moins une substance dans l'échantillon de fluide biologique, le dispositif d'analyse (130) étant adapté pour informer un opérateur du système du niveau de ladite au moins une substance, le dispositif d'analyse (130) étant en outre adapté pour informer l'opérateur afin que ce dernier sache si le niveau de ladite au moins une substance se situe à l'intérieur d'une gamme sélectionnée, et le dispositif d'analyse (130) étant en outre adapté pour être en communication avec le dispositif d'administration (120) s'agissant de la nécessité d'administrer l'agent de contraste;
dans lequel ladite au moins une substance est sélectionnée dans le groupe comprenant:
l'azote uréique du sang (BUN);
la créatinine; ou
des combinaisons de celles-ci.

2. Système d'administration selon la revendication 1, dans lequel le dispositif d'analyse (130) est en outre adapté pour informer l'opérateur d'administrer l'agent de contraste si le niveau de ladite au moins une substance se situe à l'intérieur d'une gamme sélectionnée.

3. Système d'administration selon la revendication 1, dans lequel le dispositif d'analyse (130) est en outre configuré pour coopérer avec le dispositif d'administration (120) de telle sorte que le dispositif d'administration (120) soit en outre configuré pour administrer l'agent de contraste au patient si le niveau de ladite au moins une substance envoyé par le dispositif d'analyse (130) se situe à l'intérieur d'une gamme sélectionnée.

4. Système d'administration selon la revendication 1, dans lequel le dispositif d'analyse (130) comprend en outre un dispositif de test (210) qui est configuré pour être en communication fluidique avec l'échantillon de fluide biologique, de telle sorte que le dispositif de test (210) soit en outre configuré pour fournir une indication visuelle (400) afin d'informer l'opérateur du système du niveau de ladite au moins une substance par rapport à la gamme sélectionnée.

5. Système d'administration selon la revendication 1, dans lequel le dispositif d'analyse (130) comprend en outre:
un dispositif de test (210) qui est configuré pour recevoir l'échantillon de fluide biologique et pour être en communication fluidique avec celui-ci;
un dispositif d'ordinateur (220) qui est configuré pour recevoir le dispositif de test (210) et pour être engagé de façon opérationnelle avec celui-ci afin de déterminer le niveau de ladite au moins une substance dans l'échantillon de fluide biologique.

6. Système d'administration selon la revendication 5, dans lequel le dispositif de test (210) comprend en outre un réservoir d'échantillon de fluide biologique (211), au moins un agent réactif configuré pour interagir avec ladite au moins une substance et un dispositif de transmission (215) configuré pour communiquer avec le dispositif d'ordinateur (220), et dans lequel le dispositif d'ordinateur (220) est en outre configuré pour communiquer avec le dispositif de test (210) de manière à déterminer le niveau de ladite au moins une substance.

7. Système d'administration selon la revendication 6, dans lequel le dispositif d'ordinateur (220) comprend un outre un écran d'affichage (221) qui est configuré pour fournir une indication visuelle de manière à informer l'opérateur du système du niveau de ladite au moins une substance.

8. Système d'administration selon la revendication 6, dans lequel le dispositif d'ordinateur (220) comprend un outre un dispositif d'entrée (223) qui est configuré pour recevoir des données sélectionnées dans le groupe comprenant:
des données relatives au patient;
des données relatives à la gamme sélectionnée; et
des combinaisons de celles-ci.

9. Système d'administration selon la revendication 1, 2 ou 4 à 8, et comprenant en outre un dispositif d'imagerie médicale (110) qui est configuré pour fournir une image du patient en utilisant un agent de contraste administré au patient.

10. Système d'administration selon la revendication 9 lorsqu'elle dépend de la revendication 1, et dans lequel le dispositif d'administration (120) est en outre configuré pour administrer l'agent de contraste au patient si le niveau de ladite au moins une substance envoyé par le dispositif d'analyse (130) se situe à l'intérieur de la gamme sélectionnée.

11. Système d'administration selon la revendication 9 lorsqu'elle dépend de la revendication 5, dans lequel le dispositif d'ordinateur (220) est configuré de manière à être capable de calculer un débit de filtration glomérulaire en utilisant les données reçues et le niveau de ladite au moins une substance.

12. Procédé pour évaluer la fonction rénale d'un patient avant l'administration d'un agent de contraste dans le cadre d'une procédure d'imagerie médicale, le procédé comprenant les étapes suivantes exécutées par un dispositif d'analyse (130) situé dans une procédure d'imagerie médicale (100):
recevoir un échantillon de fluide biologique provenant d'un patient;
déterminer un niveau d'au moins une substance dans l'échantillon de fluide biologique du patient;
comparer le niveau de ladite au moins une substance avec une gamme sélectionnée de niveaux de ladite au moins une substance;
informer un opérateur du dispositif d'analyse (130) afin que ce dernier sache si le niveau se situe à l'intérieur de la gamme sélectionnée; et
communiquer avec un dispositif d'administration s'agissant de la nécessité d'administrer ledit agent de contraste,
dans lequel l'étape de détermination comprend en outre la détermination d'un niveau d'une substance dans l'échantillon de fluide biologique, la substance étant sélectionnée dans le groupe comprenant:
l'azote uréique du sang (BUN);
la créatinine; ou
des combinaisons de celles-ci.

13. Produit de programme d'ordinateur capable de commander un dispositif d'analyse (130) et un dispositif d'administration (120) situés dans une procédure d'imagerie médicale (100) afin d'évaluer la fonction rénale d'un patient avant l'administration d'un agent de contraste dans le cadre d'une procédure d'imagerie médicale, le produit de programme d'ordinateur comprenant un support de stockage lisible par un ordinateur contenant des parties de code de programme lisibles par un ordinateur stockées sur celui-ci, les parties de code de programme lisibles par un ordinateur comprenant:
une partie exécutable pour déterminer un niveau d'au moins une substance dans un l'échantillon de fluide biologique prélevé chez un patient en utilisant le dispositif d'analyse (130);
une partie exécutable pour comparer le niveau de ladite au moins une substance avec une gamme sélectionnée de niveaux de ladite au moins une substance en utilisant le dispositif d'analyse (130);
une partie exécutable pour informer un opérateur du dispositif d'analyse afin que ce dernier sache si le niveau se situe à l'intérieur de la gamme sélectionnée; et
une partie exécutable pour entraîner ledit dispositif d'analyse (130) à communiquer avec ledit dispositif d'administration s'agissant de la nécessité d'administrer ledit agent agent de contraste,
dans lequel ladite au moins une substance est sélectionnée dans le groupe comprenant:
l'azote uréique du sang (BUN);
la créatinine; ou
des combinaisons de celles-ci.

14. Produit de programme d'ordinateur selon la revendication 13, comprenant en outre une partie exécutable pour administrer l'agent de contraste au patient en utilisant le dispositif d'administration (120) si le niveau de ladite au moins une substance se situe à l'intérieur de la gamme sélectionnée, de telle sorte qu'une fonction rénale sensiblement normale du patient soit dépiste avant l'administration de l'agent de contraste.

15. Produit de programme d'ordinateur selon la revendication 13, comprenant en outre une partie exécutable pour ajuster une propriété de l'agent de contraste en utilisant le dispositif d'administration (120) pour créer un agent de contraste altéré si le niveau de ladite au moins une substance se situe à l'extérieur de la gamme sélectionnée, de telle sorte que l'agent de contraste altéré puisse être éliminé par la fonction rénale du patient après l'administration de l'agent de contraste altéré.

16. Produit de programme d'ordinateur selon la revendication 15, dans lequel la propriété de l'agent de contraste est sélectionnée dans le groupe comprenant:
le volume de l'agent de contraste;
le débit de l'agent de contraste;
la concentration de l'agent de contraste;
le type de l'agent de contraste; ou
des combinaisons de celles-ci.
